(19) Europäisches Patentamt
European Patent Office
Office européen des brevets

(11) **EP 3 017 292 B1**

(12) **EUROPÄISCHE PATENTSCHRIFT**

(45) Veröffentlichungstag und Bekanntmachung des Hinweises auf die Patenterteilung:
**06.09.2023** **Patentblatt 2023/36**

(21) Anmeldenummer: **14734498.0**

(22) Anmeldetag: **02.07.2014**

(51) Internationale Patentklassifikation (IPC):
**G01N 21/31** *(2006.01)* **G01N 21/59** *(2006.01)*
**G01N 21/03** *(2006.01)*

(52) Gemeinsame Patentklassifikation (CPC):
**G01N 21/31; G01N 21/59;** G01N 2021/0364;
G01N 2021/3129

(86) Internationale Anmeldenummer:
**PCT/EP2014/064111**

(87) Internationale Veröffentlichungsnummer:
**WO 2015/000987 (08.01.2015 Gazette 2015/01)**

(54) **VORRICHTUNG UND VERFAHREN ZUR ERMITTLUNG DER KONZENTRATION EINES STOFFES IN EINEM FLEXIBLEN BEHÄLTER**

DEVICE AND METHOD FOR DETERMINING THE CONCENTRATION OF A SUBSTANCE IN A FLEXIBLE CONTAINER

DISPOSITIF ET PROCÉDÉ DE DÉTERMINATION DE LA CONCENTRATION D'UNE SUBSTANCE DANS UN CONTENANT FLEXIBLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.07.2013 DE 102013011495**

(43) Veröffentlichungstag der Anmeldung:
**11.05.2016 Patentblatt 2016/19**

(73) Patentinhaber: **Maco Pharma**
**59420 Mouvaux (FR)**

(72) Erfinder:
• **HELFMANN, Jürgen**
**14532 Klein-Machnow (DE)**
• **GERSONDE, Ingo**
**10437 Berlin (DE)**
• **CAPPIUS, Hans-Joachim**
**12157 Berlin (DE)**
• **LIEBOLD, Karsten**
**14974 Ludwigsfelde (DE)**
• **NETZ, Uwe**
**10717 Berlin (DE)**

(74) Vertreter: **Herrou, Nathalie**
**Maco Pharma**
**Pôle Propriété Industrielle**
**Rue Lorthiois**
**59420 Mouvaux (FR)**

(56) Entgegenhaltungen:
WO-A1-93/06456      WO-A1-2006/058741
US-A- 5 750 998      US-A1- 2002 167 667

**Beschreibung**

Die Erfindung hat zur Aufgabe, die Konzentration eines Inhaltsstoffes in einem Behältnis mit flexibler Wandung auf optischem Wege zu ermitteln, ohne dass die optischen Eigenschaften der Wandungen gesondert erfasst werden müssen.

Stand der Technik

Häufig besteht die Notwendigkeit, Parameter wie die Konzentration von einem oder mehreren Inhaltsstoffen von Proben bzw. Fluiden in abgeschlossenen Behältnissen zu bestimmen, ohne die Behältnisse zu öffnen und Flüssigkeiten zu entnehmen. Hierzu zählen Fluide, die steril eingeschlossen sind oder Fluide die eine starke Reaktion auf die umgebenden Milieus zeigen.

Haben die Behältnisse teilweise transparente Wandungen, bieten sich hierzu spektroskopische Verfahren an, die die Absorption und/oder Streuung des Inhaltes nutzen um die gewünschten Parameter zu ermitteln. Üblicherweise erfolgen Messungen zur Bestimmung der Konzentration bei zwei Wellenlängen - eine, die stark von der Konzentration des Inhaltsstoffes abhängig ist und eine zweite die nur schwach von der Konzentration des Inhaltsstoffes abhängig ist und der Korrektur sonstiger auftretenden Schwächungen dient. Weitere zu bestimmende Inhaltsstoffe erfordern dann weitere Wellenlängen. Dies funktioniert gut bei identischen Messbedingungen, also wenn z.B. die Behältnisse eine gleichartige optische Schwächung zeigen.

Diese Verfahren werden aber ungenau, wenn die relevanten optischen Eigenschaften der Wandung des jeweiligen Behältnisses unbekannt sind, weil Material, Wanddicke und Oberflächenstruktur von einer Messsituation zur anderen variieren.

Ein weiteres Problem tritt auf, wenn die Behältnisse oder die Inhaltsstoffe optische Strahlung streuen. Vorbekannt sind Verfahren zur Durchleuchtung von Fluiden und Feststoffen, die aufgrund der Länge des Durchstrahlungsweges, der Kenntnis der Eingangsintensität und der molekularen Extinktion des Inhaltsstoffes dessen Konzentration aus der Messung der Ausgangsintensität bestimmen können (Lambert-Beersches Gesetz). Solche Verfahren stoßen an ihre Grenzen, wenn die Streuung der Behälterwandung oder die Streuung der Inhaltsstoffe (konzentrationsabhängig) ähnlich groß werden wie die Absorption der eingestrahlten optischen Strahlung.

Lösung bieten hier Verfahren mit Erfassung von nicht absorbierten Strahlungsanteilen, z.B. quer zur Ausbreitungsrichtung, um die mehrfach gestreuten Anteile zu ermitteln.

Eine weitere vorbekannte Lösung für im Strahlweg auftretende Mehrfachstreuung ist auch, die Strahlungsausbreitung mittels theoretischen Annahmen zu simulieren (z.B. Monte-Carlo-Simulation). Dies setzt aber ebenfalls voraus, dass die gemessenen Eigenschaften der zu messenden Proben nur von den Konzentrationsunterschieden der Inhaltsstoffe hervorgerufen werden, bzw. die optischen Eigenschaften der Behälterwandung vorbekannt sind.

Ebenfalls kann bei vorbekannten optischen Eigenschaften der Behälterwandung eine Kalibration anhand Messungen mit vorbestimmten Konzentrationen der Inhaltsstoffe erfolgen. Hierbei führt eine Änderung der Mess-Situation - also der Absorption und/oder Streuung der Behälterwandung bei der verwendeten Strahlung - zu Fehlern bei der Bestimmung der Konzentration des Inhaltsstoffes.

Diese Verfahren werden klassischerweise bei wenig Streuung in den Durchstrahlungswegen in Transmissionsanordnung gemacht, wobei die Probe zwischen Lichtquelle und Detektor positioniert wird. Eine Alternative bei stärker streuenden Proben ist die Remissionsmessung, wo die Durchstrahlungswege zwischen den auf gleicher Seite der Probe befindlichen Lichtquelle und Detektor durch Streuung ein von der Streuung bestimmtes Volumen durchdringen.

Alle diese Verfahren versagen, sofern die Fluide nicht in Behältnisse mit gleichen Eigenschaften der Wandung zur Verfügung gestellt werden bzw. umgefüllt werden können, weil z.B. die Behältnisse bzw. Fluide von verschiedenen Herstellern stammen.

Die DE 603 12 737 T2 beschreibt ein aus der Streulichtmessung abgeleitetes Verfahren, worin eine Vorrichtung und ein Verfahren beschrieben wird zur Erfassung von mindestens zweimal gestreutem Licht mit mindestens einer LED als Quelle (als "Gruppe" um den Umfang angeordnet) und einem oder mehreren Photodioden als Empfänger (gleichermaßen als "Gruppe" um den Umfang angeordnet). Dies kann durch einen entlang der Schlauchlänge weiter entfernt angeordneten Kranz von Detektoren ergänzt werden. Durch die Erfassung der Streuung bei Infrarotlicht ohne direkte Bestrahlung von LED auf den Detektor wird der Hämatokrit an einem Zuleitungsschlauch zu einer Dialysevorrichtung durch Einspannen des Schlauches ermittelt. Um die Situation von der Kalibrierung - die nicht erwähnt wird, aber notwendig ist - her beherrschbar zu halten, wird die Schlauchleitung geklemmt, also die runde Schlauchgeometrie in eine zu Lichtquelle und Detektor plane Oberfläche gewandelt. Die Erfassung erfolgt mit mindestens zwei Lichtwegen, d.h. einer LED und mindestens zwei versetzt angeordneten Photodioden.

Ebenfalls wird in der DE 698 35 142 T2 eine Lösung für die Vermessung an Plasma-Beutelsystemen beschrieben, wobei eine automatisiert bestückbare Schlauchhalterung beschrieben wird, an der mit Lichtwellenleitern in Transmissions- oder Reflexionsanordnung eine breitbandige Lichtquelle und ein Spektrometer angeschlossen sind, wobei die Strahlung durch eventuelle Schrift auf dem Schlauch hindurch übertragen wird. Aus der Steigung von der mindestens bei zwei Wellenlängenbereichen gemessenen Schwächung (unter Einbeziehung des Lampenspektrums aus einem Referenzzweig) werden Konzentrationen von Hämoglobin, Bilirubin, Biliverdin, Methylenblau (eingesetzt zur Virusdeaktivierung) und Trübung in Intralipid-Äquivalenten ermittelt.

Nutzt man die Remissionsmessung mit unterschiedlichem Abstand von Lichtquelle und Detektor, so kann das durchstrahlte Volumen gezielt beeinflusst und eine Wandung auf diese Art von der dahinterliegenden Probe/Fluid separiert werden. Dies versagt, wenn die Oberflächenmorphologie (Struktur) der Wandung eine Streuung bewirkt, lateral im Bereich der Messfelder verschieden ist und/oder die Dicke der Wandung mit dem Lichtweg nicht abgestimmt ist, so dass keine bzw. zu wenig Strahlungsanteile in den Inhaltsstoff eindringen.

Gewünscht wird also ein Verfahren, welches in der Lage ist, die optischen Störeinflüsse der Behältniswandung, die unbekannt, aber während der einzelnen Messung konstant sind, auf das Messergebnis bzw. die Auswertung zu minimieren.

Vorbekannt ist aus der DE 198 80 369 ein Verfahren zur nichtinvasiven in-vivo Bestimmung von Blutinhaltsstoffen mittels Messung der Lichtabsorption bei äußerer mechanischer Einwirkung auf das vermessene Körperteil, welches mit 2 Druckmodulationsfrequenzen beaufschlagt wird und durch Licht mit mindestens zwei Wellenlängen bestrahlt wird, wovon eine, aber nicht alle, im Bereich der optischen Absorption des Blutinhaltsstoffes liegt. Es werden mindestens 4 Messsignale gewonnen, die sowohl von der Einwirkung des Lichtes als auch von der Dickenänderung abhängen und aus denen die Konzentration des Blutinhaltsstoffes bestimmt wird. Hierbei wird die Dickenänderung mit harmonischen Schwingungen aufgebracht und über die Dickenmodulations-Frequenzen aus dem Messsignal isoliert und weiterverarbeitet. Es kann eine der Frequenzen Null sein - die andere sollte dann die blutgefüllten Gefäße nicht mehr komprimieren, sondern nur noch das Gewebe. Damit wird im Prinzip die "Störung" durch die Schwächung im Gewebe isoliert - also über die Weglängenänderungsfrequenz herausgefiltert - und die zu bestimmende Größe wie an Blut in einer Küvette gemessen. Als Messmethoden sind bei mindestens 2 Wellenlängen Absorptionsmessungen in Transmissionsanordnung oder Remissionsanordnung und akusto-optische Messungen genannt.

Die WO 93/06456 A1 beschreibt eine Vorrichtung und ein Verfahren zur Bestimmung der Konzentration eines Analyten in einer flüssigen Probe, z. B. der $CO_2$-Konzentration in Blut. Die Vorrichtung umfasst eine flexible Messkammer, eine Lichtquelle, die Licht einer Wellenlänge, die von dem Analyten absorbiert wird, auf die Messkammer einstrahlt und einen Detektor, der das die Messkammer durchstrahlende Licht der Lichtquelle erfasst. Der Abstand zwischen zwei einander gegenüberliegenden Wandungen der Messkammer wird derart definiert verändert, dass Messungen durch die flüssige Probe bei zwei verschiedenen Durchstrahlungsweglängen durchgeführt werden. Die vom Detektor erfassten Intensitätsschwächungen des von der Lichtquelle auf die Messkammer eingestrahlten Lichts bei den beiden Durchstrahlungsweglängen werden unter Verwendung des Lambert-Beer-Gesetzes ausgewertet, um die Konzentration des Analyten zu bestimmen.

Erfindungsgemäße Lösung

Die erfindungsgemäße Lösung der Aufgabenstellung besteht in der Durchführung geeigneter spektroskopischer Messungen bei unterschiedlichen Durchstrahlungsweglängen durch das zu analysierende Medium. Durch die Flexibilität des Behältnisses ist es möglich, eine Transmissionsmessung durch das Behältnis hindurch so vorzunehmen, dass Messergebnisse in Abhängigkeit von mehreren unterschiedlichen Durchstrahlungsweglängen aufgenommen werden können. Dabei kann die Variation des Abstandes sowohl von Hand als auch motorisch automatisiert realisiert werden. Es ist sowohl die Auswertung bestimmter Abstände möglich als auch eine kontinuierliche Abstandsänderung bei fortlaufender Messung. Da sich die optisch relevante Geometrie der vorderen und hinteren Behälterwandung nicht ändern, wohl aber die der dazwischenliegenden Flüssigkeit, wird die dabei auftretende Änderung der Messwerte stärker von der Wechselwirkung in der dazwischen liegenden Flüssigkeit beeinflusst als von der Behälterwand. Deshalb ist durch eine geeignete Auswertung die Minimierung der Einflüsse der Behälterwandung möglich. Als Auswerteverfahren wird erfindungsgemäß eine lineare oder eine nicht-lineare Regression verwendet.

Die Aufgabenstellung wird durch eine Vorrichtung bzw. Messvorrichtung gemäß Anspruch 1 zur optischen nicht-invasiven Bestimmung der Konzentration von Inhaltsstoffen in einem flexiblen Behältnis mit einer Lichtquelle, mindestens einem Detektor und einer Recheneinheit gelöst. Die Lichtquelle ist ausgebildet auf das flexible Behältnis mit Licht einzustrahlen, wodurch das flexible Behältnis von dem Licht durchstrahlt wird. Der mindestens eine Detektor ist ausgebildet je zu erfassender Konzentration bei mindestens einer Wellenlänge oder einem Wellenlängenbereich die Erfassung der Intensitätsschwächung der verwendeten Strahlung mit unterschiedlichen Durchstrahlungsweglängen durchzuführen. Die Recheneinheit ist ausgebildet einen Einfluss der konkret vorliegenden Behälterwandung durch Quotientenbildung der Messungen verschiedener Dicken zu eliminieren. Es sind im Wesentlichen zwei Konfigurationen für die Lichtquelle und den mindestens einen Detektor der Vorrichtung möglich. In einer ersten Konfiguration kann die Vorrichtung eine Lichtquelle aufweisen, die mit vorbestimmten Wellenlängen oder vorbestimmten Wellenlängenbereichen auf das flexible Behältnis einstrahlt, beispielsweise eine Leuchtdiode oder mehrere Leuchtdioden. In diesem Fall ist der mindestens eine Detektor ausgebildet diese vorbestimmten Wellenlängen oder vorbestimmten Wellenlängenbereiche zu detektieren. Der mindestens eine Detektor kann hierfür beispielsweise ein Wellenlängen-integrierender Sensor sein. In der zweiten Konfiguration kann die Vorrichtung eine breitbandige Lichtquelle aufweisen, die mit einem kontinuierlichen Spektrum auf das flexible Behältnis einstrahlt. In diesem Fall ist der mindestens eine Detektor zur wellenlängenaufgelösten Detektion ausgebildet, beispielsweise mit einem oder mehreren Spektrometern, Monochromatoren, oder Filtern. Die beiden vorgenannten Konfigurationen können sich auch ergänzen bzw. kombiniert werden. So kann beispielsweise auch eine an einer Lichtquelle einstellbare Wellenlänge auf das flexible Behältnis eingestrahlt werden, die dann auf einen auf bestimmte Wellenlängen einstellbaren Detektor treffen, d.h., in diesem Fall können sowohl die Lichtquelle oder Lichtquellen, als auch der Detektor oder die Detektoren auf vorbestimmte Wellenlängen oder Wellenlängenbereiche eingestellt sein. Es können daher auch mehrere Lichtquellen und Detektoren parallel angeordnet sein, um Konzentrationen von Inhaltsstoffen parallel zu erfassen bzw. zu bestimmen.

Detailliertere Beschreibung der Erfindung

Hinsichtlich der Messvorrichtung kommt sowohl die Einstrahlung kontinuierlicher Spektren mit breitbandigen Lichtquellen in Frage als auch die Einstrahlung bei wenigen ausgewählten Wellenlängen, die zum Beispiel mit verschiedenen Leuchtdioden realisiert werden. Die Detektion erfolgt im ersten Fall wellenlängenaufgelöst mit Spektrometer, Monochromator oder Filter. Bei der Einstrahlung weniger Wellenlängen bzw. geeigneter Wellenlängenbereiche kann ein Wellenlängen-integrierender Sensor genutzt werden.

Die zu ermittelnde Konzentration muss sich für die optische Erfassung (durch Spektroskopie) eignen, d.h. es muss Wellenlängen oder Wellenlängenbereiche geben, bei denen eine Schwächung der Strahlung von der/den zu ermittelnden Konzentration(en) der/des Inhaltsstoffe(s) abhängt.

Der Behälter muss über mindestens einen in diesem Wellenlängenbereich transparenten Bereich verfügen; bei Transmissionsmessung müssen es mindestens zwei gegenüberliegende Bereiche sein.

Für eine Remissionsmessung muss der transparente Bereich ausreichend groß dimensioniert sein, damit die Remission ortsaufgelöst mit Strahlausbreitung durch die Wandung bis in den Bereich der Inhaltsstoffe erfolgt. Dies ist abhängig von der Dicke und Streuung der Wandung und der Streuung und Absorption der Inhaltsstoffe des Behältnisses. Die Wandung muss über diesen Messbereich ausreichend gleichartige optische Eigenschaften aufweisen.

In einer Ausgestaltung ist die Lichtquelle zu dem mindestens einen Detektor so angeordnet, dass die detektierte Strahlung unterschiedlich lange Wege durch die Inhaltsstoffe im Behältnis zurücklegt.

Dabei muss für jede zu detektierende Konzentration der Inhaltsstoffe mindestens eine Wellenlänge auswertbar sein. Wenn unbekannte Schwächungen von anderen Inhaltsstoffen ausgehen ist mindestens eine weitere Wellenlänge notwendig.

Die Messung erfolgt als Intensitätsmessung bei mindestens zwei Durchstrahlungsweglängen und für jeweils die notwendigen Wellenlängen oder Wellenlängenbereiche. Hierbei wird a priori nur eine Kalibrierung mit unterschiedlichen Wandungen der Behältnisse zwischen der zu ermittelnden Konzentration und der Intensität benötigt. Diese Auswahl der Wandungen muss aber nicht vollständig sein.

Hilfreich ist es, die Charakteristik des Messaufbaus (z.B. Schwächung infolge dickenabhängig veränderter Überstrahlung der Detektorfläche) zu kennen. Üblicherweise wird für die genaue Bestimmung der Konzentration(en) der/des Inhaltstoffe(s) mit dem verwendeten Detektor eine Dunkelmessung (ohne Beleuchtung) durchgeführt, um den Signaluntergrund zu bestimmen, welcher von den ermittelten Messwerten abgezogen werden kann.

Damit werden systematische Fehler verringert und Genauigkeiten der Konzentrationsermittlung verbessert.

Kenntnisse über die Wandung der Behältnisse sind für die konkrete Messung nicht erforderlich. Die Wandung muss natürlich durchlässig für die verwendeten Wellenlängen bzw. Wellenlängenbereiche sein, da sonst keine Strahlung bis zu den Inhaltstoffen vordringt.

Eine Messung der Intensität der Lichtquelle bei der notwendigen Wellenlänge oder dem Wellenlängenbereich vor der Messung (ohne Probe) ermöglicht eine Normierung und somit eine Vergleichbarkeit verschiedener Messungen. Eine Annahme der Intensität der Lichtquelle (anstelle einer Messung) bei der verwendeten Wellenlänge bzw. dem Integral über den verwendeten Wellenlängenbereich kann eine Messung ersetzen, jedoch mit geringerer Aussagesicherheit in Bezug auf die Auswertung.

Die Messung kann ebenfalls eine mehrfache Referenzmessung der Strahlungsintensität der Lichtquelle umfassen, um Schwankungen der Lichtquelle zu erfassen und auszugleichen.

Die Auswertung der Messdaten erfolgt durch eine Antwortfunktion R, die aus den Messgrößen (geschwächte Intensität bei einer Dicke und Wellenlänge oder einem Wellenlängenbereich $I_x(\lambda_1, d_x)$, Dicke des durchstrahlten Behältnisses $d_x$) die zu ermittelnden Konzentrationen berechnet. Hierbei steht der Index x für eine nicht näher festgelegte Zahl von Messungen (mindestens zwei) und $\lambda_1$ für eine Wellenlänge oder einen Wellenlängenbereich, bei der/dem die zu ermittelnde(n) Konzentration(en) des Inhaltsstoffes auswertbar ist/sind. Der Weg, wie diese Antwortfunktion R festgelegt wird, ist wie folgt:

Wird eine gegenüber der Durchstrahlungsweglänge breitflächige Einstrahlungs- und Detektionsfläche gewählt, kann ein eindimensionales Strahlungs-Transportmodell zugrunde gelegt werden. Hierbei ist im Wesentlichen die Winkelverteilung der Strahlung bei Durchtritt durch die Wandungen und durch alle Inhaltsstoffe wichtig. Diese ist für die Messung möglichst konstant zu gestalten. Dies kann durch eine ausreichend hohe Streuung bzw. Dicke der Durchstrahlungsweglänge durch die Inhaltsstoffe erfolgen oder durch eine entsprechende Wahl der Einstrahlungs- und Detektionsfläche. Somit kann neben der breitflächigen Einstrahlungs- und Detektionsfläche auch eine Mindest-Schichtdicke des Fluids mit den Inhaltsstoffen vorgesehen werden.

In einer Ausgestaltung der Vorrichtung ist die Lichtquelle für eine breitflächige Einstrahlung ausgebildet und der Detektor für eine breitflächige Detektion ausgebildet.

Die gemessene abgeschwächte Intensität $I_x(\lambda_1)$ setzt sich zusammen aus einem Produkt der Beiträge von erster Wand (W), zweiter Wand (W'), die auch identisch mit erster Wand sein kann, und Probe/Inhaltsstoff (P), also: W·P(d)·W'. Für die Auswertung werden für die mindestens zwei zu messenden Dicken des Behältnisses $d_1$ und $d_2$ bzw. $d_x$ die Messwerte aufeinander bezogen, d.h. dividiert. Damit ergibt sich, da sich die Beiträge der Wand W bzw. W' nicht mit der Dickenänderung ändern:

$$Q_{x1}(\lambda_1) = \frac{W \cdot W' \cdot P(d_x)}{W \cdot W' \cdot P(d_1)} = \frac{P(d_x)}{P(d_1)}$$

Mehr als eine Messgröße mit der Behältnisdicke $d_x$ (x > 1) können zu unterschiedlichen Zeitpunkten ermittelt werden, um die Genauigkeit zu erhöhen.

Diese somit wegfallenden Beiträge W und W' der Wandung auf die Schwächung haben, obwohl sie mitgemessen werden, überraschenderweise keinen nennenswerten Einfluss auf die Genauigkeit der ermittelten Konzentrationen der Inhaltsstoffe.

Die Antwortfunktion R wird erstellt, indem die zu ermittelnden Konzentrationen der Inhaltsstoffe mit den Messwerten in einer Kalibrationsmessung vermessen werden und entsprechend dem Stand der Technik über eine lineare bzw. nicht-lineare Regression unter erfindungsgemäßer Einbeziehung der Quotientenbildung entsprechende Vorhersagefunktionen gebildet werden. Dies erfolgt im Falle größerer Streubeiträge in der Schwächung eher mit nicht-linearen Regressionsmodellen, da ein linearer Zusammenhang nicht notwendig gegeben ist.

In einer Ausgestaltung ist die Vorrichtung ausgebildet Blutparameter, beispielsweise den Hämoglobingehalt von Blut zu bestimmen, das in einer verschlossenen Blutkonserve angeordnet ist. Die Vorrichtung weist eine mechanische Anordnung auf, die ausgebildet ist einen Abstand von zwei Wandungen der Blutkonserve im Bereich eines Messfeldes während der Messung definiert zu verändern.

Eine bevorzugte Anwendung der Erfindung ist daher die Bestimmung von Blutparametern an verschlossenen Blutkonserven, die nach einer Öffnung bzw. Entnahme wegen der Kontaminationsgefahr nicht mehr infundiert werden dürfen. Die als Behältnis verwendeten Blutbeutel weisen herstellerabhängig Unterschiede im Material, Dicke und in der Oberflächenstruktur auf, die sich auf die ansonsten für die Bestimmung der relevanten Blutparameter sehr geeignete Spektroskopie stark auswirken.

Mit Hilfe der mechanischen Anordnung, die den Abstand der beiden Wandungen des Blutbeutels im Bereich eines Messfeldes während der Messung definiert verändert, können Blutparameter wie zum Beispiel der Hämoglobingehalt bestimmt werden. Aus der DE 698 28 825 T2 ist zum Beispiel ersichtlich, dass es kommerzielle Geräte gibt, bei denen eine solche Bestimmung wünschenswert ist; das Volumen mit sichergestellter Konzentration kann auch durch eine Gesamtmenge Hämoglobin ausgedrückt werden.

Die Erfindung findet sich des Weiteren in der Verwendung der Vorrichtung zur Bestimmung von Blutparametern von Blut, das in einer verschlossenen Blutkonserve angeordnet ist.

Eine weitere bevorzugte Anwendung ist der Einsatz in Einweg-Bioreaktoren, wie z.B. der Wave Bioreactor von GE Healthcare und die Biostat Cultibag RM von Sartorius Stedim Biotech, sowie die flexiblen Beutel-(Bag) Systeme von S.U.B., die Biostat CultiBag STR und der XDR Bioreactor und andere. Das Messen und Regeln eines Zellkulturprozesses in einem Einwegbioreaktor stellt eine Herausforderung dar, da der Kunststoffbeutel in dem die Kultivierung stattfindet ein geschlossenes, sterilisiertes System ist. Die üblichen Sensoren zur Prozessführung, wie Thermostatfühler, pH- und Leitfähigkeitsmesselektroden, Glucose- und Sauerstoffelektroden, Drucksensoren, etc. können nicht einfach bei Bedarf in den Beutel eingeführt werden, sondern müssen bereits in den sterilen Beutel integriert sein. Daraus ergeben sich Probleme, weil die Behältnisse einerseits trocken hergestellt, gelagert und ausgeliefert werden und andererseits weitere Kalibrierungen vor der Benutzung des Beutels nicht möglich sind. Auch muss bei der Herstellung der Systeme entschieden werden, welche Konfiguration der möglichen Sensoren eingebaut werden soll. Der Einsatz optischer Sensoren kann diese Probleme umgehen, da z.B. einfach zu integrierende Sensormaterialien (optische Indikatormaterialien) kostengünstig eingebaut werden können und bei Bedarf mit der notwenigen Auslesetechnik genutzt werden. Üblicherweise zählt zu den zu ermittelnden Größen die Zellzahl, die durch ihre mit dem Wachstum zunehmende Trübung eine einfache Ermittlung über Transmissionsmessungen erschwert. Hier kann eine dickenabhängige Messung unter Vernachlässigung der Einflüsse der Behältniswandung große Vorteile bringen.

In einer Ausgestaltung weist die Vorrichtung Sensormaterialien und Auslesetechnik zur Messung und Regelung eines Zellkulturprozesses in einem Einwegbioreaktor auf.

Weitere bevorzugte Anwendungen ergeben sich aus Einweg-Prozess-Durchführungen, die ebenfalls auf eine Kapselung der durchgeführten Verfahren in Kunststoff-Behältnissen basieren. Hier sind ähnliche Einsatzmöglichkeiten denkbar, wie bei den Einweg-Bioreaktoren.

Neben den im Umfeld der Biotechnologie angesiedelten Anwendungen zur biotechnologischen Herstellung vor allem chemischer Verbindungen kommt auch der Einsatz in der Lebensmitteltechnologie in Betracht, z.B. für die alkoholische Gärung in Beuteln, die in große Tanks eingelegt werden, um Reinigungsaufwand und Kontamination zu verringern, wo die Fernhaltung des Sauerstoffs ein weiteres wesentliches Ziel ist.

Die Erfindung findet sich auch in einer Verwendung der Vorrichtung in der Lebensmitteltechnologie.

In einer Ausgestaltung ist die Vorrichtung ausgebildet die Einstrahlung immer durch eine so stark streuende Schichtdicke durchzuführen, dass bei den genutzten Durchstrahlungsweglängen durch die Dickenänderung keine Änderung der Streuwinkelverteilung am Detektor erfolgt. Durch die Einstrahlung wird das flexible Behältnis durchstrahlt. Im Inneren des flexiblen Behältnisses, d.h. zwischen den Wandungen des Behältnisses, kann die Schichtdicke durch Verformung der Wandung geändert werden. Somit ist es möglich die Durchstrahlungsweglängen - also die Weglängen des Lichts durch das zu analysierende Medium und damit die Inhaltsstoffe in dem Behältnis - durch Dickenänderungen der Schichtdicke im Inneren des Behältnisses zu ändern.

Die Erfindung findet sich auch in einem Verfahren zur optischen nicht-invasiven Bestimmung der Konzentration von Inhaltsstoffen in einem flexiblen Behältnis gemäß Anspruch 4.

Das Verfahren umfasst einen Schritt des Einstrahlens auf das flexible Behältnis mit Licht. Das Verfahren umfasst des Weiteren einen Schritt des Erfassens einer Intensitätsschwächung der verwendeten Strahlung mit unterschiedlichen Durchstrahlungsweglängen bei mindestens einer Wellenlänge oder einem Wellenlängenbereich je zu erfassendem Parameter. Des Weiteren umfasst das Verfahren einen Schritt des Quotientenbildens der Messungen verschiedener Dicken der Behälterwandung, um einen Einfluss der konkret vorliegenden Behälterwandung zu eliminieren.

In einer Ausgestaltung des Verfahrens werden eine breitflächige Einstrahlung und eine breitflächige Detektion durchgeführt.

In einer Ausgestaltung des Verfahrens erfolgt die Einstrahlung immer durch eine so stark streuende Schichtdicke, dass bei den genutzten Durchstrahlungsweglängen durch die Dickenänderung keine Änderung der Streuwinkelverteilung am Detektor erfolgt.

Die Erfindung findet sich auch in einer Verwendung des Verfahrens zur Bestimmung von Blutparametern von Blut, das in einer verschlossenen Blutkonserve angeordnet ist.

Die Erfindung findet sich des Weiteren in einer Verwendung der Vorrichtung zum Ausführen wenigstens eines Teils der Schritte des Verfahrens.

Beschreibung der Zeichnungen

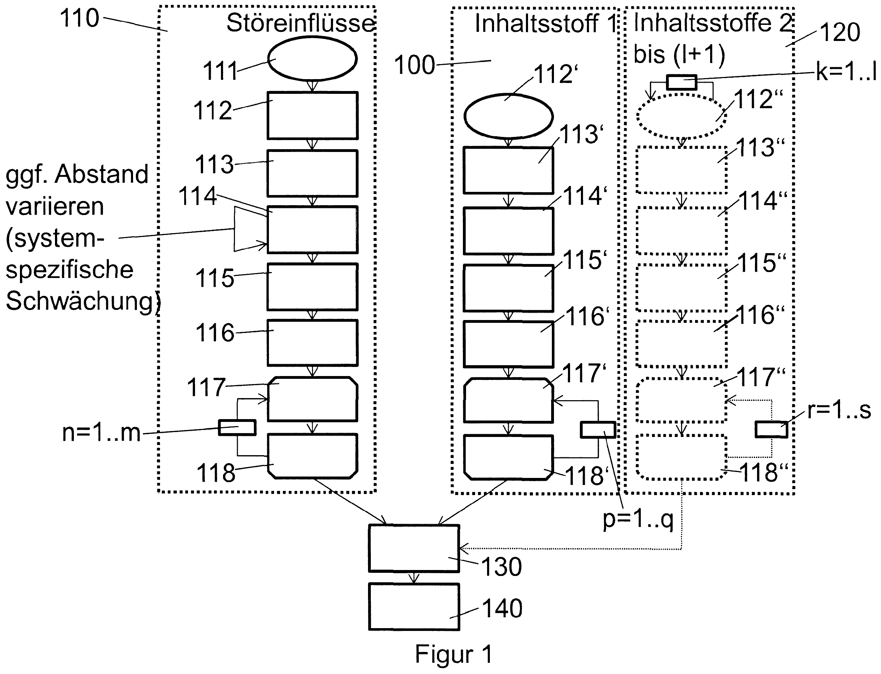

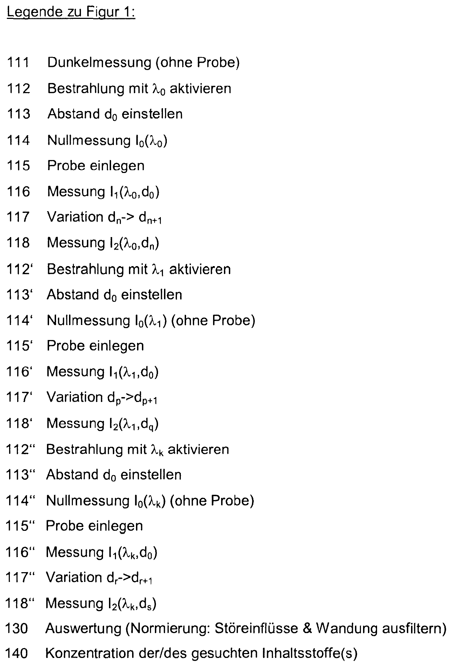

In Figur 1 ist ein *Ablauf der Erfassung der Messwerte* dargestellt. Für einen minimal erforderlichen Ablauf ist die in der Mitte dargestellte Abfolge 100 durchzuführen. Mit Hilfe der dargestellten Abfolge 100 kann eine Konzentration eines Inhaltsstoffes erfasst werden. Unter dem Begriff Erfassen der Konzentration ist hier das Bestimmen eines Wertes der Konzentration zu verstehen.

Wird eine verbesserte Genauigkeit benötigt, müssen die Schritte der links dargestellten Abfolge 110(Störeinflüsse) durchgeführt werden. Zur Erfassung weiterer Konzentrationen ist die rechts dargestellte Abfolge 120 für jede weitere Konzentration durchzuführen. Es können auch Konzentrationen für andere Inhaltsstoffe erfasst werden. Dies ist möglich indem die einzustrahlenden Wellenlängen entsprechend den Konzentrationen der anderen Inhaltsstoffe angepasst werden. Beispielsweise können Konzentrationen von verschiedenen Inhaltsstoffen eines flexiblen Behältnisses erfasst werden. Somit können also mit dem dargestellten Verfahren Konzentrationen mehrerer Inhaltsstoffe erfasst werden.

Im Folgenden werden die Schritte der Abfolge 110 erläutert.

In einem Schritt 111 ist im Einzelnen für die Erfassung der Störeinflüsse die Messung der Dunkelsignale des Detektors bei ausgeschalteter Beleuchtung und ggf. mit oder ohne Probe im Strahlengang zu ermitteln. Im Schritt 112 wird eine Bestrahlung mit einer Wellenlänge oder einem Wellenlängenbereich $\lambda_0$ durch den für alle Messungen gemeinsamen Durchstrahlungsweg durch die Probe geschickt, also einer Wellenlänge, die von unbekannten Schwächungen stärker absorbiert wird, als von den zu ermittelnden Parametern der Inhaltsstoffe. In der Regel wird die Wellenlänge oder der Wellenlängenbereich $\lambda_0$ so gewählt, dass sie/er in der Nähe der auszuwertenden $\lambda_1$ - bzw. $\lambda_2$ ff. - liegen, um eine Korrektur der veränderten Streuwinkelverteilung durch die Messung bei $\lambda_0$ zu ermöglichen.

Im Schritt 113 ist, die Vorrichtung auf einen Ausgangsabstand $d_0$ einzustellen und im Schritt 114 ohne Probe die Intensität der auf den Detektor treffenden Strahlung zu ermitteln. Dies dient dem Ausgleichen von dickenabhängigen systembedingten Intensitätsänderungen. So erhält man also für jede zu messende Dicke einen Bezugswert zu einer definierten Intensität, auf die später normiert werden kann, um diese Störungen zu eliminieren. Die Eliminierung der Größe kann auch durch lineare Interpolation mit nur wenigen Stützstellen einer Messgröße oder auf andere geeignete Weise erfolgen, ohne dass die erfindungsgemäße Durchführung des Verfahrens beeinflusst wird.

Im Schritt 115 wird die Probe in den Durchstrahlungsweg eingebracht, so dass im folgenden Schritt 116 eine Messung $I_2(\lambda_0, d_0)$ erfolgt, die eine Intensität bei eingelegter Probe und mit Einfluss unbekannter Schwächungen bei einer Ausgangsdicke $d_0$ erfasst. Die nächsten Schritte 117 und 118 umfassen sukzessive Veränderungen der Dicke von $d_0$ nach $d_m$ (n wird hier als Variable für die geeignete Anzahl von Zwischenschritten genutzt) (Schritt 117) mit Messungen $I_2(\lambda_0, d_n)$ (Schritt 118) um die dickenabhängigen Schwächungen als Rohwerte zu erfassen.

Für die Erfassung der Konzentration des Inhaltsstoffes ist ein ähnlicher Ablauf wie für die Störeinflüsse vorgesehen, mit dem einzigen Unterschied, dass eine andere Wellenlänge bzw. ein anderer Wellenlängenbereich $\lambda_1$ verwendet wird, die/der von der zu ermittelnden Konzentration der/des Inhaltsstoffe(s) stärker absorbiert wird, als von unbekannten Schwächungen oder anderen Parametern der/des Inhaltsstoffe(s). Den Schritten der Abfolge 110 entsprechende Schritte der Abfolge 100 sind mit einem Strich gekennzeichnet. Die Variable für die geeignete Anzahl von Zwischenschritten ist hier p genannt, die Enddicke $d_q$. Eine Übereinstimmung von n mit p (Anzahl der gemessenen Dicken) oder m mit q (Enddicke) ist möglich aber nicht zwingend.

Die Anfangsdicke oder wenn der Vorgang in umgekehrter Reihenfolge durchgeführt wird, die Enddicke sollte aber übereinstimmen. Die Reihenfolge der Schritte kann auch verändert werden.

Für die Erfassung weiterer Konzentrationen 2 bis (I+1) der Inhaltsstoffe ist ebenfalls der gleiche Ablauf vorgesehen. Den Schritten der Abfolge 110 entsprechende Schritte der Abfolge 120 sind mit zwei Strichen gekennzeichnet. Die Variable I kann 1 oder eine andere Zahl annehmen. Für jede zu ermittelnde Konzentration ist mindestens eine Wellenlänge oder ein Wellenlängenbereich $\lambda_k$ auszuwählen, die/der von der zu ermittelnden Konzentration der Inhaltsstoffe 2 bis (I+1) jeweils stärker absorbiert wird, als von unbekannten Schwächungen oder anderen Parametern der/des Inhaltsstoffe(s). Die Variable für die geeignete Anzahl von Zwischenschritten ist hier r genannt, die Enddicke $d_s$. Eine Übereinstimmung von r mit n und/oder p (Anzahl der gemessenen Dicken) oder s mit m und/oder q (Enddicke) ist möglich aber nicht zwingend.

Für die Auswertung wird in einem Schritt 130 nach den bereits beschriebenen Schritten zur Normierung, Elimination der Störeinflüsse und Wandung auf die Rohwerte der Messgrößen $I_x$ die Antwortfunktion R angewendet, deren Eingabewert die Wellenlängen oder Wellenlängenbereiche $\lambda_x$, die dazugehörigen geschwächten Intensitätswerte bei einer Wellenlänge oder einem Wellenlängenbereich und einer Dicke $I_x(\lambda_1, d_x)$ und die Dicke des durchstrahlten Behältnisses $d_x$ sind.

Erfindungsgemäß ist als Ergebnis der Auswertung die Konzentration eines Inhaltsstoffes aufgeführt (Schritt 140). Außerhalb des durch die Ansprüche definierten Schutzumfangs der Erfindung können dies auch andere Parameter der/des Inhaltsstoffe(s) sein.

Liste der Bezugzeichen

$\lambda_0$ Wellenlänge oder Wellenlängenbereich, bei der/dem eine Schwächung der Strahlung möglichst nicht von den zu ermittelnden Parametern der Inhaltsstoffe abhängt

$\lambda_1$ Wellenlänge oder Wellenlängenbereich, bei der/dem eine Schwächung der Strahlung überwiegend von dem zu ermittelnden ersten Parameter der/des Inhaltsstoffe(s) abhängt

$\lambda_k$ Wellenlänge oder Wellenlängenbereich, bei der/dem eine Schwächung der Strahlung von weiteren zu ermittelnden Parametern der/des Inhaltsstoffe(s) abhängt und möglichst nicht von den übrigen zu ermittelnden Parametern der/des Inhaltsstoffe(s) abhängt, außer diese dienen zur Verbesserung der Genauigkeit

d Durchstrahlungsweglänge

l Intensität

k Variable für die Zahl der weiteren Wellenlängen(-bereiche) zur Ermittlung weiterer Parameter der Inhaltsstoffe oder verbesserter Genauigkeit (beginnt bei 2).

l Zahl zwischen 1 und der Anzahl der weiteren Wellenlängen(-bereiche) zur Ermittlung weiterer Parameter der Inhaltsstoffe oder verbesserter Genauigkeit

n Variable für die Dickenstufung der Durchstrahlungsweglänge d bei $\lambda_0$

m Zahl zwischen 1 und der Gesamtzahl der Dickenstufungen der Durchstrahlungsweglänge d bei $\lambda_0$

p Variable für die Dickenstufung der Durchstrahlungsweglänge d bei $\lambda_1$

q Zahl zwischen 1 und der Gesamtzahl der Dickenstufungen der Durchstrahlungsweglänge d bei $\lambda_1$

r Variable für die Dickenstufung der Durchstrahlungsweglänge d bei jeder realisierten Wellenlänge oder jedem realisiertem Wellenlängenbereich $\lambda_k$

s Zahl zwischen 1 und der Gesamtzahl der Dickenstufungen der Durchstrahlungsweglänge d, ggf. unterschiedlich bei jeder realisierten Wellenlänge oder jedem realisiertem Wellenlängenbereich $\lambda_k$

x Index / Variable unbestimmten Inhalts

**Patentansprüche**

1. Vorrichtung zur optischen nicht-invasiven Bestimmung einer Konzentration von Inhaltsstoffen in einem flexiblen Behältnis, wobei die Vorrichtung umfasst

   - eine Lichtquelle, die ausgebildet ist, auf das flexible Behältnis mit Licht einzustrahlen, so dass das flexible Behältnis von dem Licht durchstrahlt wird,

- eine mechanische Anordnung, die ausgebildet ist, einen Abstand zwischen zwei Wandungen des flexiblen Behältnisses im Bereich eines Messfeldes während einer Messung definiert zu verändern, so dass eine Durchstrahlungsweglänge durch die Inhaltsstoffe im flexiblen Behältnis verändert werden kann,

- mindestens einen Detektor,

wobei der Detektor ausgebildet ist, je zu erfassender Konzentration eines Inhaltsstoffes bei mindestens einer Wellenlänge oder einem Wellenlängenbereich, welche bzw. welcher von dem Inhaltsstoff stärker absorbiert wird als von unbekannten Schwächungen, Messungen zur Erfassung einer Intensitätsschwächung des von der Lichtquelle auf das flexible Behältnis eingestrahlten und dieses durchstrahlenden Lichts mit unterschiedlichen Durchstrahlungsweglängen durch die Inhaltsstoffe im flexiblen Behältnis durchzuführen, und

- eine Recheneinheit, wobei die Recheneinheit ausgebildet ist, einen Einfluss der konkret vorliegenden Behältniswandung durch Quotientenbildung von von dem Detektor bei den unterschiedlichen Durchstrahlungsweglängen durch die Inhaltsstoffe im flexiblen Behältnis erfassten Messdaten zu eliminieren,

wobei die Recheneinheit weiter ausgebildet ist, eine Auswertung der Messdaten unter Verwendung einer Antwortfunktion R durchzuführen, die aus den vom Detektor erfassten Messdaten die zu ermittelnde Konzentration von Inhaltsstoffen berechnet,

**dadurch gekennzeichnet, dass** die Antwortfunktion R über eine lineare oder nicht-lineare Regression aus Kalibrationsmessungen unter Einbeziehung der Quotientenbildung erzeugt ist.

2. Vorrichtung nach Anspruch 1, wobei das flexible Behältnis eine verschlossene Blutkonserve ist.

3. Vorrichtung nach Anspruch 2, wobei die Vorrichtung ausgebildet ist, als die Konzentration von Inhaltstoffen den Hämoglobingehalt von Blut zu bestimmen.

4. Verfahren zur optischen nicht-invasiven Bestimmung einer Konzentration von Inhaltsstoffen in einem flexiblen Behältnis, wobei das Verfahren umfasst

- Durchstrahlen des flexiblen Behältnisses mit Licht,

- Erfassen einer Intensitätsschwächung des das flexible Behältnis durchstrahlenden Lichts mittels eines Detektors, bei mindestens einer Wellenlänge oder einem Wellenlängenbereich $\lambda_1$ je zu erfassender Konzentration eines Inhaltsstoffes, wobei die Wellenlänge oder der Wellenlängenbereich $\lambda_1$ von dem Inhaltsstoff stärker absorbiert wird als von unbekannten Schwächungen,

- Verändern einer Durchstrahlungsweglänge des Behältnisses durch Variieren eines Abstands zwischen zwei Wandungen des Behältnisses,

- wobei mittels des Detektors Messungen zur Erfassung der Intensitätsschwächung des das flexible Behältnis durchstrahlenden Lichts bei der mindestens einen Wellenlänge oder dem Wellenlängenbereich $\lambda_1$, mit unterschiedlichen Durchstrahlungsweglängen durch die Inhaltsstoffe im flexiblen Behältnis durchgeführt werden,

- Elimination eines Einflusses der konkret vorliegenden Behältniswandung durch Bilden eines Quotienten von von dem Detektor bei den unterschiedlichen Durchstrahlungsweglängen durch die Inhaltsstoffe im flexiblen Behältnis erfassten Messdaten und

- Auswerten der Messdaten unter Verwendung einer Antwortfunktion R, die aus den Messdaten die zu ermittelnde Konzentration des Inhaltsstoffes berechnet, **dadurch gekennzeichnet, dass** die Antwortfunktion R über eine lineare oder nicht-lineare Regression aus Kalibrationsmessungen unter Einbeziehung der Quotientenbildung erzeugt ist.

5. Verfahren nach Anspruch 4, wobei die Einstrahlung immer durch eine so stark streuende Schichtdicke erfolgt, dass bei den genutzten Durchstrahlungsweglängen durch die Abstandsänderung zwischen den Wandungen des Behältnisses keine Änderung der Streuwinkelverteilung am Detektor erfolgt.

6. Verfahren nach Anspruch 4 oder 5, wobei eine Bestrahlung mit einer Wellenlänge oder einem Wellenlängenbereich $\lambda_0$ durch das flexible Behältnis geschickt wird, wobei die Wellenlänge oder der Wellenlängenbereich $\lambda_0$ von unbekannten Schwächungen stärker absorbiert wird, als von dem Inhaltsstoff dessen Konzentration ermitteln werden soll.

7. Verwendung einer Vorrichtung nach Anspruch 1 zur Bestimmung von Konzentrationen von Inhaltsstoffen von Blut, das in einer verschlossenen Blutkonserve angeordnet ist.

8. Verwendung eines Verfahrens nach Anspruch 4 zur Bestimmung von Konzentrationen von Inhaltsstoffen von Blut, das in einer verschlossenen Blutkonserve angeordnet ist.

9. Verwendung einer Vorrichtung nach Anspruch 1 in der Lebensmitteltechnologie.

10. Verwendung einer Vorrichtung nach Anspruch 1 zum Ausführen wenigstens eines Teils der Schritte des Verfahrens nach Anspruch 4.

**Claims**

1. Device for the optical, non-invasive determination of a concentration of constituents in a flexible container, wherein the device comprises

   - a light source that is configured to irradiate light onto the flexible container so that light irradiates through the flexible container,
   - a mechanical arrangement that is designed to change a distance between two walls of the flexible container in the area of a measuring field during a measurement in a defined manner so that a path length of the light irradiated through the constituents in the flexible container can be changed,
   - at least one detector, wherein the detector is designed to carry out measurements to record an intensity attenuation of the light irradiated from the light source onto the flexible container and light irradiated through it with different radiation-through path lengths through the constituents in the flexible container for each concentration of a constituent to be recorded at at least one wavelength or a wavelength range which is absorbed by the constituents more strongly than by unknown attenuations,
   - a calculating unit, wherein the calculating unit is designed to eliminate an influence of the specifically present container wall through quotient formation of the measuring data recorded by the detector at the different radiation-through path lengths through the constituents in the flexible container,

   wherein the calculating unit is also designed evaluate the measuring data using a response function R which calculates the concentration of constituents to be determined from the measuring data recorded by the detector, **characterised in that** the response function R is produced by way of a linear or non-linear regression from calibration measurements with the inclusion of the quotient formation.

2. Device according to claim 1, wherein the flexible container is a unit of stored blood.

3. Device according to claim 2, wherein, as the concentration of constituents, the device is designed to determine the haemoglobin content of blood.

4. Method for the optical, non-invasive determination of a concentration of constituents in a flexible container, wherein the method comprises

   - irradiating light through the flexible container,
   - recording, by means of a detector, an intensity attenuation of the light irradiated through the flexible container at at least one wavelength or a wavelength range $\lambda_1$ for each concentration of a constituent to be recorded, wherein the wavelength or the wavelength range $\lambda_1$ is absorbed by the constituent more strongly than by unknown attenuations,
   - changing an radiation-through path length of the container by varying a distance between two walls of the container,
   - wherein, by means of the detector, measurements are carried out to record the intensity attenuation of the light irradiating through the flexible container at the at least one wavelength or the wavelength range $\lambda_1$ with different radiation-through path lengths through the constituents in the flexible container,
   - elimination of an influence of the specifically present container wall through formation of a quotient from the measuring data recorded by the detector at the different radiation-through path lengths through the constituents in the flexible container, and
   - evaluation of the measuring data using a response function R which from the measuring data calculates the concentration of the constituent to be determined, **characterised in that** the response function R is produced by way of a linear or non-linear regression from calibration measurements with the inclusion of the quotient formation.

5. Method according to claim 4, wherein light is always irradiated in through such a strongly scattering layer thickness, that at the used radiation-through path lengths, through the change in distance between the walls of the container, no change in the scattering angle distribution on the detector takes place.

6. Method according to claim 4 or 5, wherein light is irradiated through the flexible container with a wavelength or a wavelength range $\lambda_0$, wherein the wavelength or the wavelength range $\lambda_0$ is absorbed by unknown attenuations more strongly than by the constituent, the concentration of which is to be determined.

7. Use of a device according to claim 1 for determining concentrations of constituents of blood that is held in a closed unit of stored blood.

8. Use of a method according to claim 4 for determining concentrations of constituents of blood that is held in a closed unit of stored blood.

9. Use of a device according to claim 1 in food technology.

10. Use of a device according to claim 1 to for carrying out at least some of the steps of the method according to claim 4.

**Revendications**

1. Dispositif pour la détermination optique non invasive d'une concentration d'ingrédients dans un récipient souple, le dispositif comprenant

- une source lumineuse destinée à irradier de la lumière sur le récipient souple, de sorte que le récipient souple est traversé par la lumière,
- un agencement mécanique qui est conçu pour modifier d'une manière définie une distance entre deux parois du récipient souple dans la zone d'un champ de mesure lors d'une mesure, de sorte qu'une longueur de trajet de rayonnement à travers le contenu dans le récipient souple peut être modifiée,

- au moins un détecteur,
dans lequel le détecteur est conçu pour effectuer, en fonction de la concentration d'un ingrédient à mesurer, des mesures à au moins une longueur d'onde ou une plage de longueurs d'onde qui est plus absorbée par l'ingrédient que par les atténuations inconnues, afin de détecter une réduction d'intensité de la lumière irradiée par la source lumineuse sur le récipient souple et rayonnant à travers celui-ci avec différentes longueurs de trajet de rayonnement à travers le contenu dans le récipient souple, et

- - une unité de calcul, l'unité de calcul étant conçue pour éliminer une influence de la paroi spécifique du récipient en formant un quotient de données de mesure enregistrées par le détecteur pour les différentes longueurs de trajet de rayonnement à travers le contenu dans le récipient souple,

dans lequel l'unité de traitement est en outre conçue pour effectuer une évaluation des données de mesure à l'aide d'une fonction de réponse R, qui calcule la concentration d'ingrédients à déterminer à partir des données de mesure enregistrées par le détecteur,

**caractérisé en ce que** la fonction de réponse R est générée via une régression linéaire ou non linéaire à partir de mesures d'étalonnage incluant la formation du quotient.

2. Dispositif selon la revendication 1, dans lequel le récipient souple est une poche de sang scellée.

3. Dispositif selon la revendication 2, dans lequel le dispositif est conçu pour déterminer la teneur en hémoglobine du sang en tant que concentration d'ingrédients.

4. Procédé de détermination optique non invasive d'une concentration d'ingrédients dans un récipient souple, le procédé comprenant

- irradier le récipient souple avec de la lumière,
- détecter une réduction de l'intensité de la lumière traversant le récipient souple au moyen d'un détecteur, à au moins une longueur d'onde ou une gamme de longueurs d'onde $\lambda_1$ pour chaque concentration d'un ingrédient à détecter, la longueur d'onde ou la gamme de longueurs d'onde $\lambda_1$ étant plus fortement absorbée par l'ingrédient que par des atténuations inconnues,
- modifier une longueur de trajet de rayonnement du récipient en faisant varier une distance entre deux parois du récipient,
- moyennant quoi le détecteur est utilisé pour effectuer des mesures pour détecter la réduction d'intensité de la lumière irradiée à travers le récipient souple à au moins une longueur d'onde ou la plage de longueurs d'onde $\lambda_1$, avec différentes longueurs de trajet de rayonnement à travers le contenu dans le récipient souple,
- élimination d'une influence réelle de la paroi du récipient en formant un quotient de données de mesure enregistrées par le détecteur aux différentes longueurs de trajet de rayonnement à travers le contenu dans le récipient souple et
- -évaluation des données de mesure à l'aide d'une fonction de réponse R, qui calcule la concentration de l'ingrédient à déterminer à partir des données de mesure, **caractérisé en ce que** la fonction de réponse R est générée via une régression linéaire ou non linéaire à partir de mesures d'étalonnage incluant la formation du quotient.

5. Procédé selon la revendication 4, dans lequel l'irradiation a toujours lieu à travers une épaisseur de couche tellement fortement diffusante qu'avec les longueurs de trajet de rayonnement utilisées, il n'y a pas de modification dans la répartition des angles de diffusion au niveau du détecteur en raison du changement de distance entre les parois du récipient.

6. Procédé selon la revendication 4 ou 5, dans lequel une irradiation avec une longueur d'onde ou une gamme de longueurs d'onde $\lambda_0$ est envoyée à travers le récipient souple, la longueur d'onde ou la gamme de longueurs d'onde $\lambda_0$ étant plus fortement absorbée par des atténuations inconnues que par l'ingrédient dont la concentration est à déterminer.

7. Utilisation d'un dispositif selon la revendication 1 pour déterminer les concentrations de composants du sang disposés dans une poche de sang scellée.

8. Utilisation d'un procédé selon la revendication 4 pour déterminer les concentrations de composants du sang disposés dans une poche de sang scellée.

9. Utilisation d'un dispositif selon la revendication 1 en technologie alimentaire.

10. Utilisation d'un dispositif selon la revendication 1 pour réaliser au moins une partie des étapes du procédé selon la revendication 4.

EP 3 017 292 B1

Störeinflüsse

Inhaltsstoff 1

Inhaltsstoffe 2
bis (l+1)

110

111

112

113

ggf. Abstand
variieren
(systemspezifische
Schwächung)

114

115

116

117

n=1..m

118

100

112'

113'

114'

115'

116'

117'

118'

120

k=1..l

112"

113"

114"

115"

116"

117"

118"

r=1..s

p=1..q

130

140

Figur 1

Legende zu Figur 1:

111   Dunkelmessung (ohne Probe)

112   Bestrahlung mit $\lambda_0$ aktivieren

113   Abstand $d_0$ einstellen

114   Nullmessung $I_0(\lambda_0)$

115   Probe einlegen

116   Messung $I_1(\lambda_0, d_0)$

117   Variation $d_n \rightarrow d_{n+1}$

118   Messung $I_2(\lambda_0, d_n)$

112'   Bestrahlung mit $\lambda_1$ aktivieren

113'   Abstand $d_0$ einstellen

114'   Nullmessung $I_0(\lambda_1)$ (ohne Probe)

115'   Probe einlegen

116'   Messung $I_1(\lambda_1, d_0)$

117'   Variation $d_p \rightarrow d_{p+1}$

118'   Messung $I_2(\lambda_1, d_q)$

112"   Bestrahlung mit $\lambda_k$ aktivieren

113"   Abstand $d_0$ einstellen

114"   Nullmessung $I_0(\lambda_k)$ (ohne Probe)

115"   Probe einlegen

116"   Messung $I_1(\lambda_k, d_0)$

117"   Variation $d_r \rightarrow d_{r+1}$

118"   Messung $I_2(\lambda_k, d_s)$

130   Auswertung (Normierung: Störeinflüsse & Wandung ausfiltern)

140   Konzentration der/des gesuchten Inhaltsstoffe(s)

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 60312737 T2
- DE 69835142 T2
- DE 19880369
- WO 9306456 A1
- DE 69828825 T2